# EUROPÄISCHE PATENTANMELDUNG

(11) **EP 4 850 634 A2**
(43) Veröffentlichungstag der Anmeldung: **09.12.2026**
(21) Anmeldenummer: 26168269.4
(22) Anmeldetag: 26.03.2026
(51) Int. Cl.: A61F 2/16

(54) **KÜNSTLICHE KAPSELTASCHE**

(30) Priorität: 05.06.2025 DE 102025122081
(71) Anmelder: Hovsepyan, Asatur Nickolay, 0078 Yerevan (AM)
(72) Erfinder: Hovsepyan, Asatur Nickolay, 0078 Yerevan (AM)
(74) Vertreter: Keilitz & Partner Patentanwälte PartGmbB

(57) **Zusammenfassung**

Die Erfindung betrifft eine künstliche Kapseltasche zur Fixierung einer Hinterkammer-Intraokularlinse (pcIOL) (8) im menschlichen Auge bei nahezu oder vollständig fehlender Kapselunterstützung mit den in den unabhängigen Ansprüchen angegebenen Merkmalen.

## Beschreibung

### Hintergrund der Erfindung

Die Erfindung betrifft das Gebiet der Medizin und insbesondere mikrochirurgische Implantate, die in der Augenheilkunde zur intraokularen Korrektur aphakischer Augen mit fehlender oder erheblich beschädigter Kapselunterstützung verwendet werden.

Nach einer Kataraktentfernung mit Komplikationen durch einen schweren Kapseltaschenriss oder eine schwere zonulare Dehiszenz gibt es in der Regel ein Problem bei der Befestigung der Intraokularlinse (IOL). Zur Befestigung von IOLs werden daher künstliche Kapseltaschen eingesetzt, wie sie aus früheren Patentanmeldungen des Autors [1], [2] & [3] bereits bekannt sind. Als nächstkommender Stand der Technik wird die vom Autor in Armenien patentierte künstliche Kapseltasche zur Fixierung an der Sklera [3] angesehen.

Eine Aufgabe der Erfindung ist es, die natürliche Linsenkapsel in Fällen eines deutlichen Verlusts der Zonulaunterstützung und/oder bei Kapselrissen durch eine künstliche Kapseltasche zu ersetzen, die als stabile und genau zentrierte universelle Halterung für die Hinterkammer-Intraokularlinse (pcIOL) dient. Die künstliche Kapseltasche soll gleichzeitig ermöglichen, die Operationsdauer zu verkürzen sowie die Wahrscheinlichkeit der Entwicklung chirurgischer und postoperativer Komplikationen zu verringern. Darüber hinaus sollte es möglich sein, individuell berechnete IOLs (fast alle Modelle) für Patienten anstelle von speziellen monofokalen IOLs (dreiteilig - für die Yamane-Technik und vierschlaufenförmig - für die Canabrava-Technik) zu verwenden, wenn sich die Umstände bei einer Operation, z. B. aufgrund eines versehentlichen Verlusts der Kapselstütze, unerwartet ändern. Schließlich wird der ELP-Wert (effektive Linsenposition) in mehreren Berechnungsformeln basierend auf der anatomischen Position des Ziliarsulcus im Patientenauge und den Abmessungen des ACB vorhersagbar.

Gelöst wird diese Aufgabe durch die in den unabhängigen Ansprüchen angegebenen Merkmale. Weitere Ausführungen der Erfindung ergeben sich aus den Unteransprüchen.

Gemäß der Erfindung wird eine künstliche Kapseltasche zur Fixierung einer Hinterkammer-Intraokularlinse (pcIOL) im menschlichen Auge bei nahezu oder vollständig fehlender Kapselunterstützung mit speziellen Eigenschaften vorgeschlagen. Eine erste Variante der künstlichen Kapseltasche umfasst einen ringförmigen, insbesondere polygonalen, Rahmen mit mehreren Halterungen zur Befestigung der pcIOL, wobei der ringförmige Rahmen eine Rahmenebene definiert. Die künstliche Kapseltasche umfasst weiterhin mehrere Flügel, die jeweils über einen Verbindungsabschnitt, der sich senkrecht oder schräg zur Rahmenebene erstreckt, mit dem ringförmigen Rahmen verbunden sind, wobei die Flügel jeweils einen langen, drahtförmigen Abschnitt aufweisen, von dem ein kurzer Fußabschnitt abzweigt. Die langen, drahtförmigen Abschnitte der Flügel zeigen gemäß einer bevorzugten Ausführungsform alle in die gleiche Umfangsrichtung, also entweder in Uhrzeigerrichtung oder entgegen der Uhrzeigerrichtung. Die vorstehend genannte Umfangsrichtung bezieht sich dabei auf eine Achse, die senkrecht auf die Rahmenebene steht.

Die langen, drahtförmigen Abschnitte der Flügel sind vorzugsweise bogenförmig gekrümmt.

Die langen, drahtförmigen Abschnitte der Flügel sind vorzugsweise als ein einfacher Draht ausgeführt.

Die langen, drahtförmigen Abschnitte der Flügel erstrecken sich vorzugsweise in einer zur Rahmenebene parallelen Ebene.

In einer bevorzugten Ausführungsform der Erfindung haben die langen, drahtförmigen Abschnitte jeweils ein freies Ende. Die freien Enden zeigen in Umfangsrichtung betrachtet vorzugsweise jeweils in eine erste Richtung, wohingegen die kurzen Fußabschnitte in die entgegengesetzte Umfangsrichtung zeigen.

Der zentrale ringförmige Rahmen und die Flügel liegen im Wesentlichen in parallelen Ebenen. Der Abstand der Ebenen wird dabei durch die Länge und Ausrichtung der Verbindungsabschnitte bestimmt.

In einer bevorzugten Ausführungsform umfasst die künstliche Kapseltasche drei am zentralen Rahmen befestigte Flügel.

Die Position, an der die kurzen Fußabschnitte von den drahtförmigen Abschnitten abzweigen, liegt vorzugsweise näher am Rahmen als am freien Ende der drahtförmigen Abschnitte.

Eine zweite Variante einer künstlichen Kapseltasche zur Fixierung einer Hinterkammer-Intraokularlinse (pcIOL) im menschlichen Auge bei nahezu oder vollständig fehlender Kapselunterstützung, umfasst wiederum einen ringförmigen Rahmen mit mehreren Halterungen zur Befestigung der pcIOL, wobei der Rahmen eine Rahmenebene definiert. Die zweite Variante umfasst außerdem mehrere, vorzugsweise zwei, Flügel, die jeweils über einen Verbindungsabschnitt, der sich senkrecht oder schräg zur Rahmenebene erstreckt, mit dem ringförmigen, insbesondere polygonalen, Rahmen verbunden sind. Im Unterschied zur ersten Variante haben die Flügel jeweils auf der Seite des Rahmens ein Fußende, das sich im weiteren Verlauf nach außen in zwei gleiche drahtförmige Abschnitte aufteilt, die symmetrisch gegenüberliegend angeordnet sind. Die Symmetrieachse ist vorzugsweise eine in Radialrichtung verlaufende Achse. Die beiden drahtförmigen Abschnitte eines Flügels zeigen bei der zweiten Variante in entgegengesetzte Richtungen. Sie sind zwischen dem Fußende, genauer ab dem Punkt, an dem sie sich gabeln, und ihrem jeweiligen freien Ende bogenförmig gekrümmt. Die Krümmung der drahtförmigen Abschnitte ist vorzugsweise konvex nach außen.

Die drahtförmigen Abschnitte sind vorzugsweise jeweils als ein einfacher Draht ausgeführt.

Die zwei drahtförmigen Abschnitte eines Flügels sind vorzugsweise gleich lang.

Zusammenfassend besteht ein Aspekt der Erfindung darin, dass ein einteiliger künstlicher Beutel aus einem Polymer (z. B. Polyvinylidenfluorid, PVDF) mit mechanischem (Form-) Gedächtnis so konstruiert ist, dass er im geöffneten Zustand mehrere Flügel und einen zentralen ringförmigen Rahmen (kreisförmig, sechseckig oder achteckig usw.) mit IOL-Halterungen aufweist. Darüber hinaus ist bei der ersten Variante auf einer Seite jedes Flügels ein Stützbein symmetrisch geformt, das im Ziliarsulcus aufliegt. Die vordere Hälfte jedes Flügels hat die Form einer haptikartigen langen, drahtförmigen Schnur (die Dicke muss etwa dem Durchmesser eines 5-0 oder 6-0 Prolene-Nahtfadens entsprechen), die durch die Sklera geführt und durch das Schmelzen ihrer Spitze fixiert wird, um einen Flansch zum Halten des gesamten Komplexes zu erhalten (Yamane, [4], Canabrava [5]). Darüber hinaus fällt die Ebene der Flügel mit der Ebene des Ziliarsulcus des Patientenauges zusammen, die Ebene des zentralen Rahmens befindet sich jedoch in der Hinterkammer. Der ELP-Wert (effektive Linsenposition) wird durch die anatomische Position des Ziliarsulcus im Auge des Patienten und die Länge der Verbindungsabschnitte des ACB bestimmt, die die Flügel mit dem zentralen Rahmen verbinden. Daher können wir mehrere ACB-Größen herstellen, um die endgültige Entscheidung des Chirurgen darüber sicherzustellen, wie viel tiefer die ELP von der Ebene des Ziliarsulcus aus liegen muss. Beachten Sie, dass der Durchmesser des virtuellen Kreises, der alle Stützbeine der Flügel umreißt, ungefähr dem Durchmesser des Ziliarsulcus des Patientenauges entsprechen muss. Daher müssen bei der Herstellung der ACB alle möglichen Größen (z. B. 12,5 mm, 13,0 mm, 13,5 mm, 14,0 mm usw.) berücksichtigt werden, die dem Chirurgen zur Verfügung stehen, um die beste Wahl basierend auf den per anteriorer OCT/oder UBM gemessenen Daten des jeweiligen Patientenauges treffen zu können. Darüber hinaus ermöglicht der Satz verschiedener Längen der Verbindungsbalken (z. B. 2,0 mm, 2,5 mm, 3,0 mm) für jede ACB-Größe den Chirurgen, die endgültige Position der pcIOL nahe dem ELP-Wert der Berechnungsdaten zu erreichen.

Der erfindungsgemäße ACB ist dank der Vorhersagbarkeit des ELP-Werts auch nützlich, wenn eine Kapselunterstützung vorhanden ist, jedoch ein Risiko besteht, die zonuläre Unterstützung zu schwächen (z. B. bei einem Pseudoexfoliationssyndrom, oder wenn der Patient wahrscheinlich zukünftige Sportverletzungen erleidet). Insbesondere bei der Planung von Premium-IOLs kann der Chirurg die passende ACB auswählen, eine solche teure IOL innerhalb der ACB im natürlichen Linsenkapselsack fixieren und zusätzliche Sklera-Stützpunkte für langfristige Stabilität hinzufügen.

### DETAILLIERTE BESCHREIBUNG

Das Wesen der Erfindung wird durch die Zeichnungen verdeutlicht. Es zeigen:
Fig. 1 eine Ansicht einer künstlichen Kapseltasche im geöffneten Zustand von vorne;
Fig. 2 eine Ansicht derselben von der Seite;
Fig. 3 eine Ansicht derselben von oben;
Fig. 4 die endgültige Position der künstlichen Kapseltasche im Augapfel mit der Hinterkammer-IOL-Halterung (Vorderansicht);
Fig. 5 eine Ansicht eines Augapfels mit eingesetzter Kapseltasche von der Seite, wobei die Hälfte der Augapfelwand zur besseren Visualisierung entfernt und der Kreis des Ziliarsulcus als gestrichelte Linie dargestellt ist;
Fig. 6 eine zweite Ausführungsform einer künstlichen Kapseltasche entsprechend den Figuren 1 bis 5, aber mit versetzten Positionen der IOL-Halterungen am Zentralrahmen;
Fig. 7 eine künstliche Kapseltasche im gefalteten Zustand, verpackt in einer Implantationskartusche vor dem Einsetzen in das Patientenauge;
Fig. 8 eine erste Phase der Implantation einer künstlichen Kapseltasche, wobei die künstliche Kapseltasche mithilfe einer Kartusche in die vordere Kammer eingeführt wird, so dass die distalen Flügel und der zentrale Rahmen auf der Iris aufliegen und der drahtförmige Abschnitt des proximalen Arms außerhalb der Wunde bleibt;
Fig. 9 eine zweite Phase der Implantation der künstlichen Kapseltasche, wobei mit Hilfe einer Mikropinzette die Spitze des langen, drahtförmigen Abschnitts jedes Flügels des ACB in das Lumen der Nadel eingeführt und zur Außenfläche des Augapfels herausgeführt wird;
Fig. 10 eine weitere Phase der Implantation der künstlichen Kapseltasche, wobei der letzte drahtförmige Abschnitt auf die gleiche Weise wie in Fig. 9 eingeführt und zur Außenfläche des Augapfels hinausgeführt wird, während alle anderen langen drahtförmigen Flügel auf der Außenfläche des Augapfels verbleiben und der zentrale Rahmen seine Position hinter der Iris einnimmt;
Fig. 11 eine weitere Phase der Implantation der künstlichen Kapseltasche nach der Externalisierung aller drahtförmigen Abschnitte der Flügel (wie in Fig. 9 und 10 gezeigt) und der endgültigen Zentrierung des ACB, wobei die überschüssigen Teile der drahtförmigen Abschnitte abgeschnitten werden, während sie mit einer Mikropinzette in der Nähe der Sklera festgehalten werden;
Fig. 12 die endgültige Position der eingesetzten künstlichen Kapseltasche;
Fig. 13 eine Phase, in der die IOL platziert wird, wobei die pcIOL mit Hilfe eines Mikrospatels an die Halterungen des Zentralrahmens herangeführt wird;
Fig. 14-26 jeweils dieselbe Ansicht wie die Figuren 1-13 für eine künstliche Kapseltasche in einer 4-Flügel-Version;
Fig. 27 eine Ansicht einer spiegelverkehrten Version des ACB für Linkshänder.

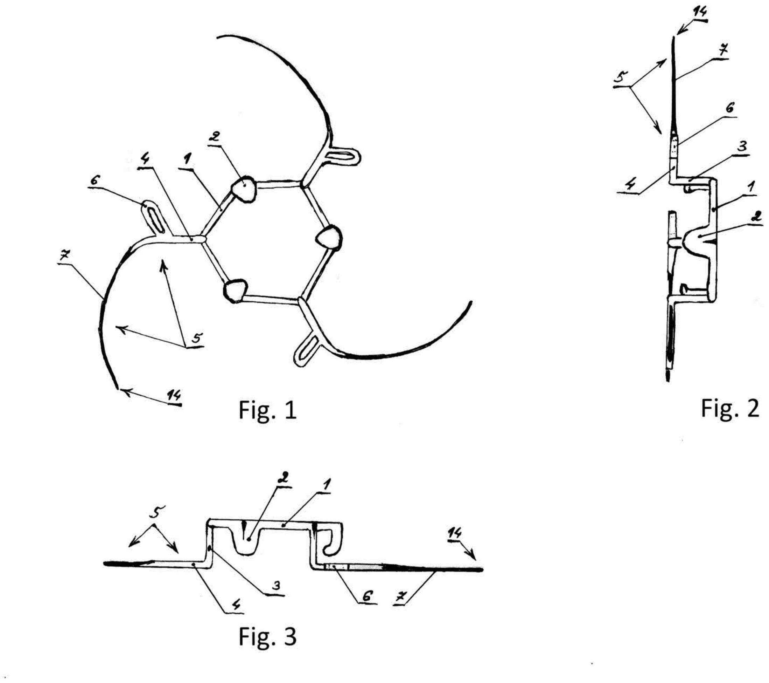

Die Figuren 1 bis 3 zeigen verschiedene Ansichten einer künstlichen Kapseltasche (ACB) aus einem drahtartigen, flexiblen Material. Die ACB ist vorzugsweise aus einem Kunststoff, wie z. B. einem monolithischen Polymer, insbesondere Polypropylen (PP), hergestellt.

In der in den Figuren 1-3 dargestellten Ausführungsform hat die ACB einen zentralen, ringförmigen Rahmen 1, an dem mehrere Halterungen 2 zur Befestigung einer IOL-Optik 8 ausgebildet sind. Am Rahmen 1 sind außerdem mehrere Flügel 5 befestigt, die sich vom Rahmen 1 in radialer Richtung nach außen erstrecken und über Verbindungsabschnitte 3 mit dem Rahmen 1 verbunden sind. Die Verbindungsabschnitte 3 verlaufen dabei senkrecht zur Rahmenebene. Der Rahmen 1 und die Flügel 5 liegen im Wesentlichen in parallelen Ebenen. Die Position der Halterungen 2 befindet sich in Umfangsrichtung betrachtet jeweils in der Mitte zwischen zwei benachbarten Flügeln 5.

In der dargestellten Ausführungsform ist der Rahmen 1 als ein hexagonaler Ring ausgeführt, an dem drei Flügel 5 im Abstand von jeweils 120 Grad angeordnet sind. Jeder Flügel 5 umfasst einen langen, drahtförmigen Abschnitt 7 und einen verhältnismäßig kürzeren Fußabschnitt 6, der vom langen, drahtförmigen Abschnitt 7 abzweigt. Die langen Abschnitte 7 sind jeweils bogenförmig gekrümmt.

Im dargestellten Ausführungsbeispiel verlaufen die langen, drahtförmigen Abschnitte 7 entgegen dem Uhrzeigersinn, wohingegen die Fußabschnitte 6 jeweils in Uhrzeigersinn zeigen. Die Position, an der die Fußabschnitte 6 von den drahtförmigen Abschnitten 7 abzweigen, liegt vorzugsweise näher am Rahmen 1, insbesondere bei etwa einem Drittel der Gesamtlänge der drahtförmigen Abschnitte 7. Die Länge der Fußabschnitte 6 ist kürzer als die Länge der langen, drahtförmige Abschnitte 7.

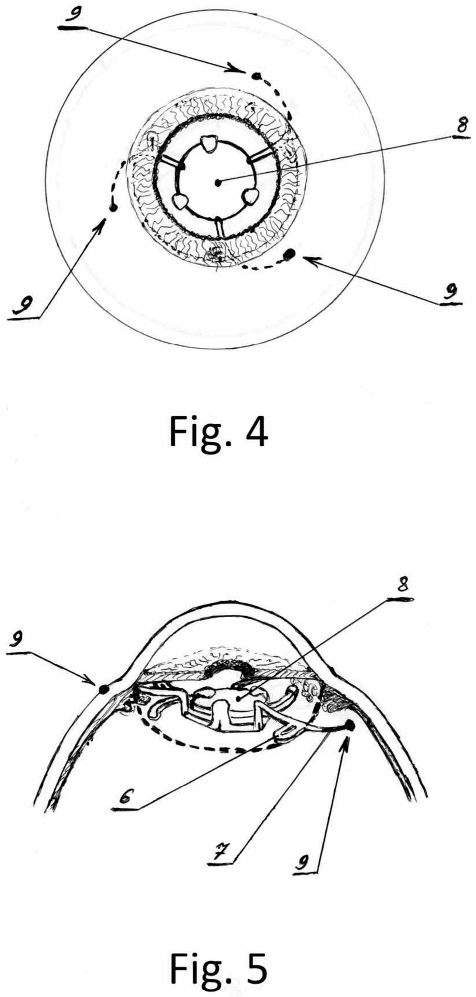

Die Fig. 4 und 5 zeigen eine ACB, die in den vorderen Abschnitt eines menschlichen Auges implantiert ist. Der optische Teil der pcIOL 8 wird von den Halterungen 2 in einer vorgesehenen Tiefe in der hinteren Augenkammer fixiert. Der kurze Fußabschnitt 6 jedes Flügels 5 liegt in der Ziliarfurche - die Position ist als gestrichelter Kreis markiert. Der lange, drahtförmige Abschnitt 7 verläuft durch die Schichten der Sklera. Zur besseren Visualisierung ist der proximale Sektor der Augapfelwand in Fig. 5 abgeschnitten, während der ACB-Flügel 5 und der gestrichelte Kreis der Ziliarfurche in diesem Sektor verbleiben.

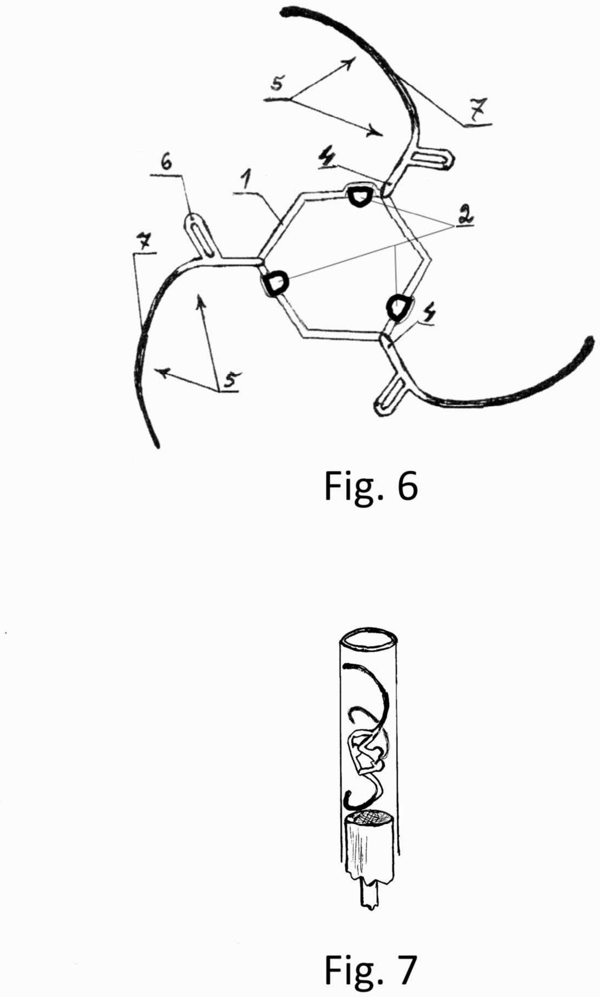

Fig. 6 zeigt eine ähnliche Ausführungsform wie Fig. 1 mit einem hexagonalen Rahmen 1, wobei sich aber die Halterungen 2 für die IOL-Optik 8 nicht in der Mitte zwischen zwei benachbarten Flügeln 5, sondern jeweils näher am Fußende 4 eines der Flügel 5 als am Fußende 4 eines benachbarten Flügels 5 befinden. Diese Variante des ACB ist besonders hilfreich, wenn für das Auge des Patienten eine torische (sphärozylindrische) pcIOL geplant ist, da sie eine Feinausrichtung der pcIOL ermöglicht.

Die künstliche Kapseltasche ist derart ausgelegt, dass sie zwischen einem zusammengeklappten Zustand, in dem sie klein zusammengefaltet ist, und einem ausgefalteten Zustand, in dem sie eine größere Fläche einschließt, transformierbar ist. In Fig. 7 befindet sich die künstliche Kapseltasche in einem gefalteten Zustand innerhalb einer Implantationskartusche.

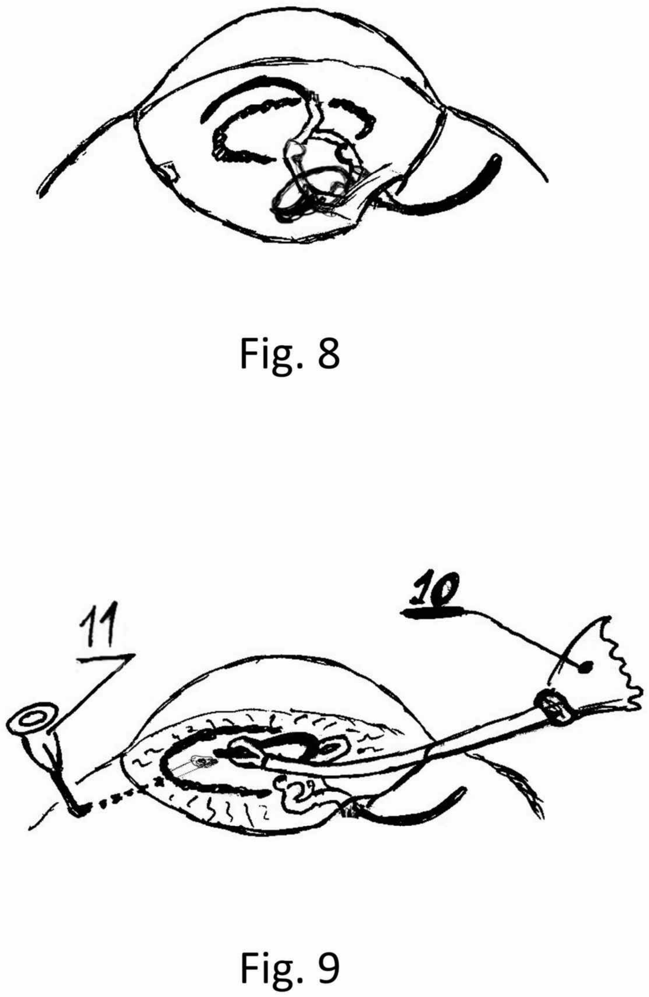

Die Fig. 8 bis 11 zeigen verschiedene Zustände während der Implantation der ACB in das Auge eines Patienten. Der Chirurg drückt zunächst den Kartuschen-Injektor langsam nach vorne, wodurch die künstliche Kapseltasche in die Vorderkammer eingeführt wird und sich entfaltet. In dem in Fig. 8 dargestellten Zustand liegen die distalen Flügel 5 der künstlichen Kapseltasche auf der Iris, während ein proximaler, drahtförmiger Abschnitt 7 außerhalb der Wunde verbleibt.

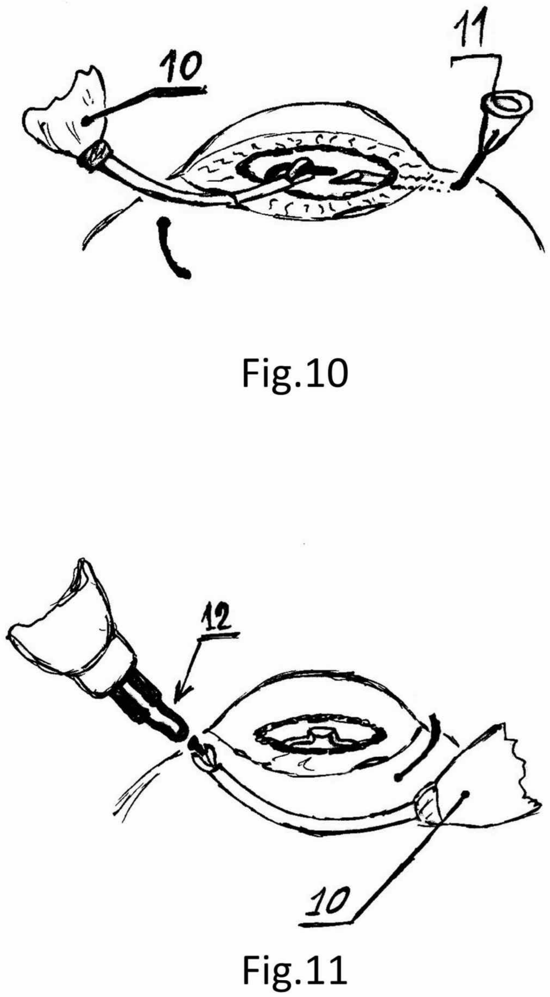

In den Fig. 9 und 10 (ähnlich der Yamane-Methode) wird mit Hilfe einer Mikropinzette 10 die Spitze des drahtförmigen Abschnitts 7 des proximalen Flügels 5 in das Lumen einer Nadel 11 geschoben und durch die Schichten der Sklera nach außen geführt. Die verbleibenden drahtförmigen Abschnitte 7 werden auf die gleiche Weise vorbereitet. In Fig. 11 werden schließlich die überschüssigen Teile der drahtförmigen Abschnitte 7 abgeschnitten, während sie mit der Mikropinzette 10 nahe der Sklera festgehalten werden. Die Spitzen der drahtförmigen Abschnitte 7 werden dann mittels eines Thermokauters 12 geschmolzen und verdickt, um einen Flansch 9 zu erhalten.

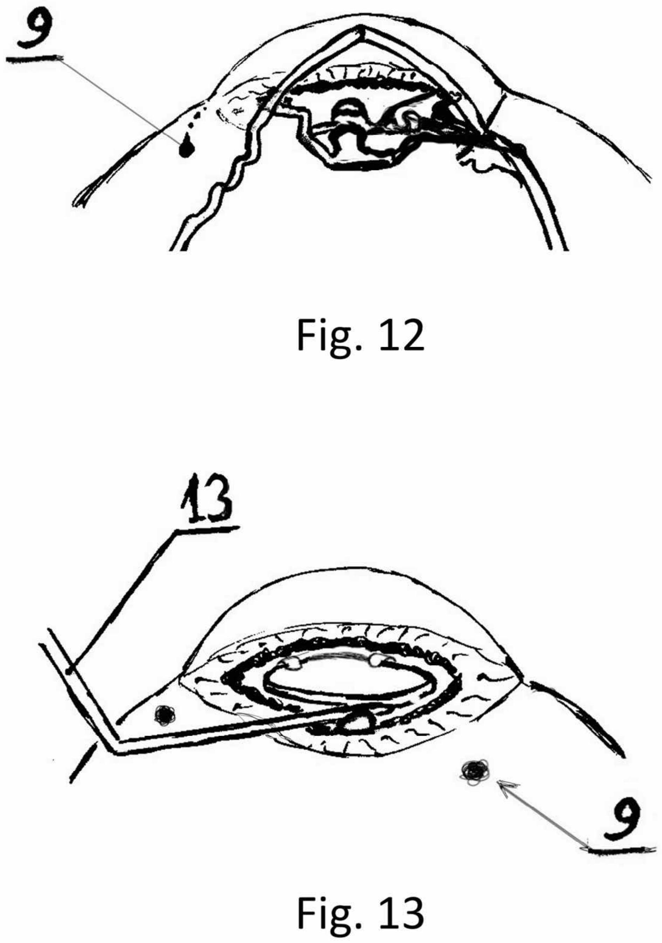

In dem in Fig. 12 dargestellten Zustand befindet sich die künstliche Kapseltasche an ihrem endgültigen Platz, wobei sie durch die Flansche 9 in der Sklera fixiert ist.

Fig. 13 zeigt das Einführen der pcIOL 8 in die bereits eingesetzte Kapseltasche. Dabei wird die letzte Halterung 12 mit einem Mikrospatel 13 an die pcIOL 8 gelegt.

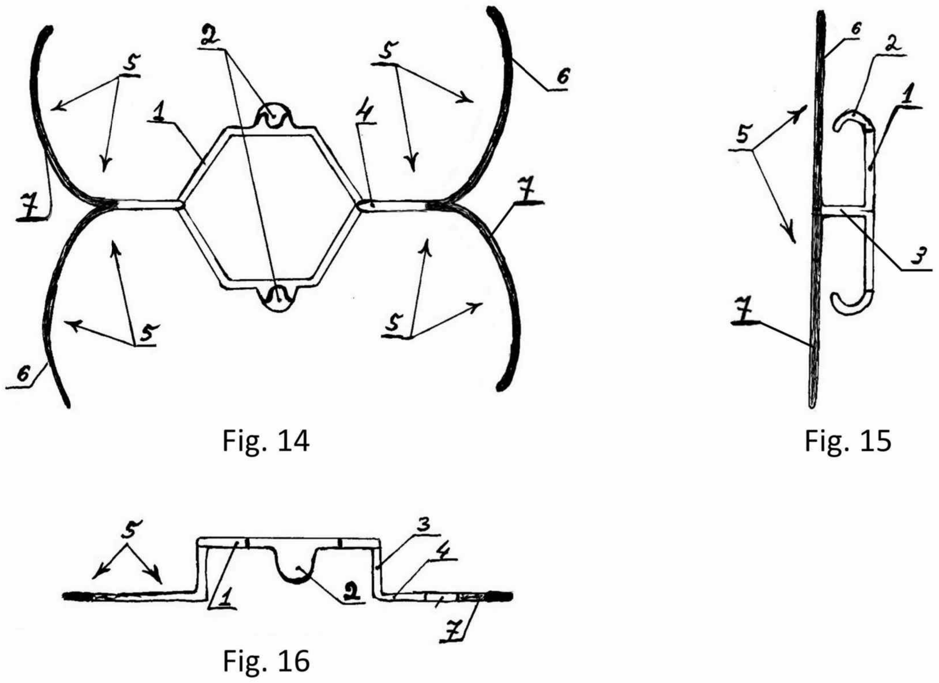

Die Figuren 14 bis 16 zeigen eine zweite Variante einer ACB mit zwei Flügeln 5, die jeweils über eine Verbindungsabschnitt 3 mit dem zentralen Rahmen 1 verbunden sind. Jeder Flügel 5 hat ein Fußende 4, das sich in zwei drahtförmige Abschnitte 7 aufteilt, die spiegelsymmetrisch gegenüberliegend angeordnet sind. Die freien Enden 14 der drahtförmigen Abschnitte 7 eines Flügels 5 weisen in entgegengesetzte Richtungen.

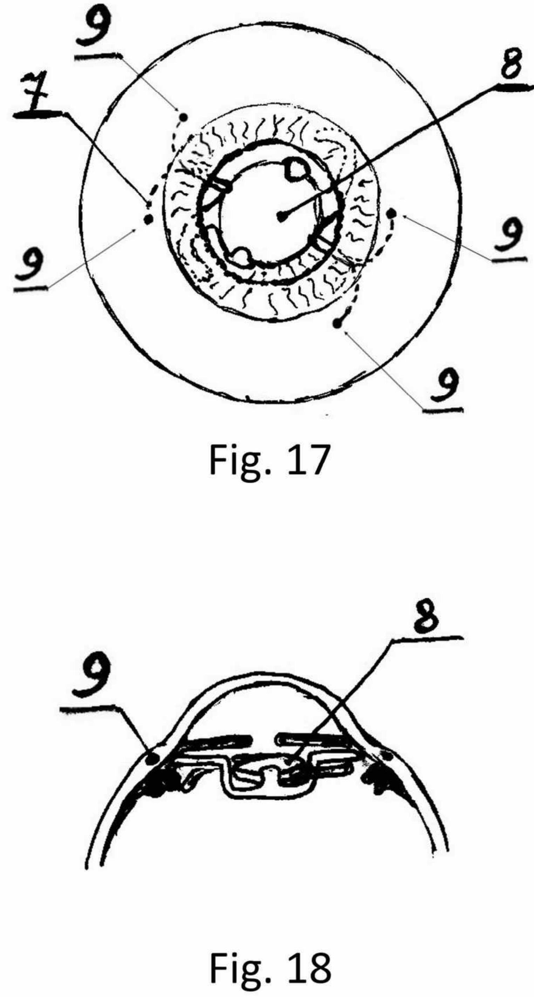

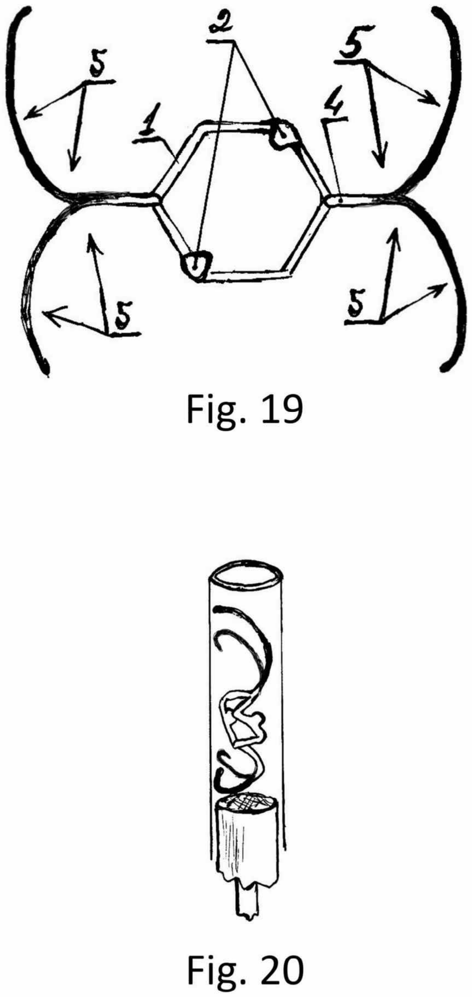

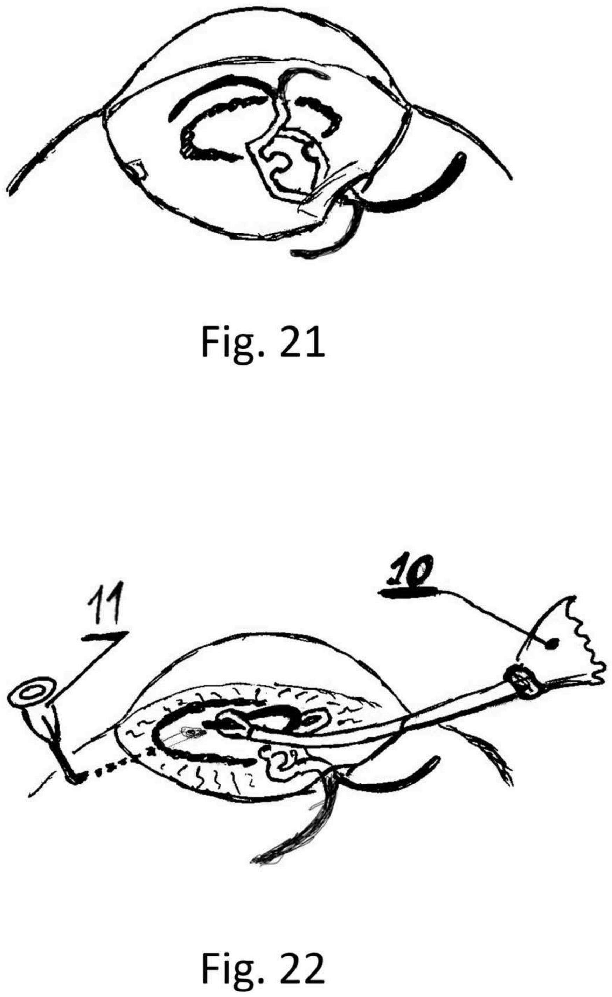

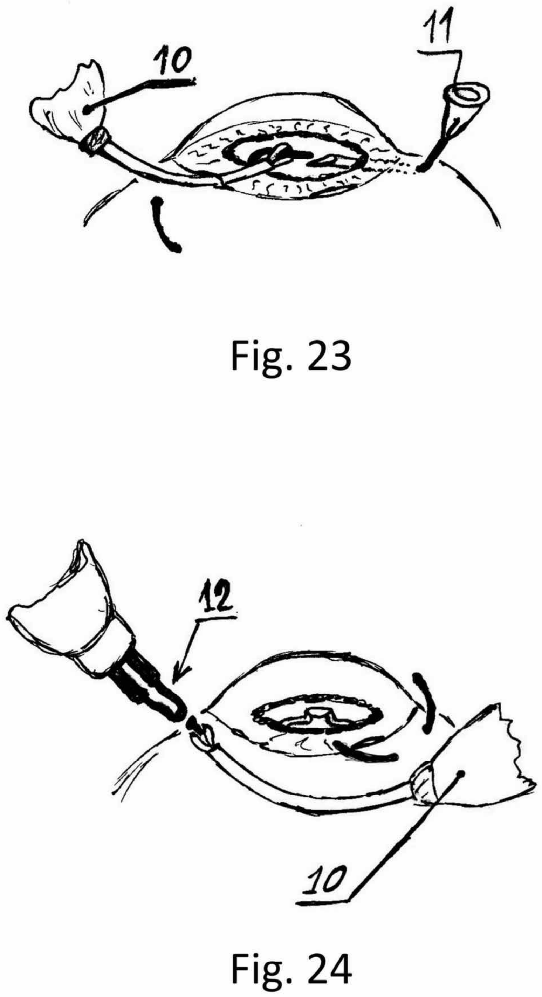

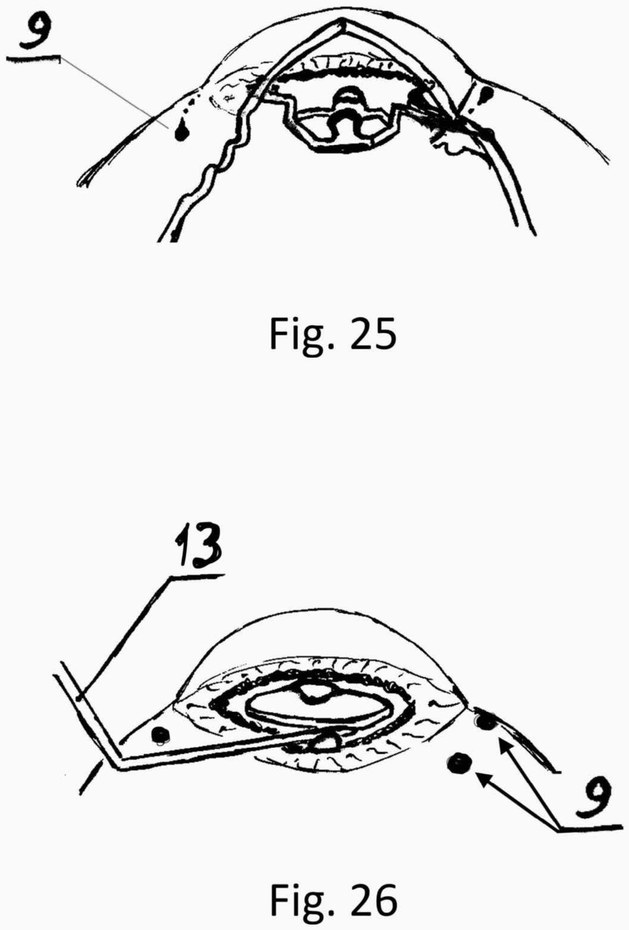

Die Figuren 17 bis 26 entsprechen jeweils der Darstellung der Figuren 4 bis 13, aber in Bezug auf die zweite Variante der künstlichen Kapseltasche.

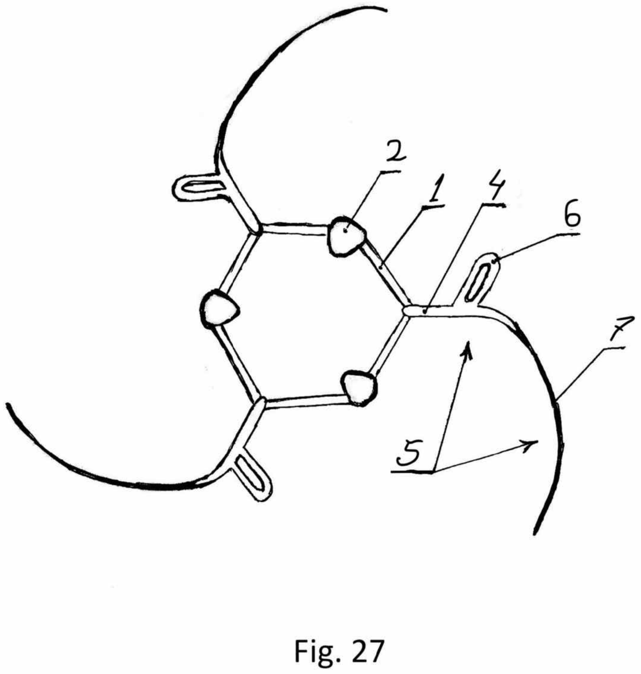

Fig. 27 zeigt eine Ausführungsform einer ACB, die nach dem Prinzip der in den Figuren 1-3 gezeigten ersten Variante aufgebaut ist, aber für Linkshänder ausgelegt ist. In diesem Fall haben die langen, drahtförmigen Abschnitte 7 Enden 14, die im Uhrzeigersinn ausgerichtet sind.

Die hier vorgeschlagene künstliche Kapseltasche ermöglicht die Verwendung fast aller Arten von pcIOLs (mit Ausnahme des lamellaren Designs von Pierce) und ihre optimale Positionierung auf Höhe der Knotenpunkte der natürlichen Linse bei gleichzeitiger Gewährleistung von Stabilität, Feinzentrierung und genauer Fokussierung, da die ELP nicht geschätzt, sondern streng auf Grundlage des Abstands von der Ebene des Ziliarsulcus des Patientenauges berechnet wird. Mit anderen Worten: Der Abstand zwischen der Ebene der Flügel der vorgeschlagenen künstlichen Kapseltasche und der zentralen Rahmenebene kann einen signifikanten Wert für die IOL-Berechnungsformeln darstellen. Dank dieser Eigenschaften können bei der Operation schwieriger Kataraktfälle mit erheblichem oder vollständigem Verlust der Kapselunterstützung unerwünschte Komplikationen im Zusammenhang mit der Fixierung der Intraokularlinse vermieden werden, wie z. B. Keratopathie, Reibung der hinteren Oberfläche der Iris, die zum UGH-Syndrom führt, Pupillendeformationen durch die Erfassung der IOL-Optik und allmähliche Dezentrierung der IOL-Position bis hin zu ihrem Absinken in den Glaskörperraum.

Die vorgeschlagene künstliche Kapseltasche ist eine Variante der vom Autor vorgeschlagenen künstlichen Kapselsäcke zur Irisfixierung [1], [2] und Sklerafixierung [3]. Sie bietet den Vorteil einer 3- oder 4-Punkt-Flanschfixierung mit der Sklera, die eine bessere Zentrierung und eine länger anhaltende Stabilität der pclOL-Position im Augapfel ermöglicht. Die 4-Punkt-Fixierungstechnik von Sergio Canabrava ist bekannt und bietet mehr Stabilität, da die IOL-Ebene nicht verkippt wird. Die 2-Punkt-Fixierungstechnik nach Yamane [4] ist mit der 4-Punkt-Canabrava-Technik [5] vergleichbar.

Abschließend weist der Autor darauf hin, dass Augenchirurgen weltweit (die die Canabrava-Technik bereits beherrschen) die 4-Punkt-Version der vorgeschlagenen künstlichen Kapseltasche zunächst schneller beherrschen werden, während die Anwendung der künstlichen Kapseltasche mit 3 Flügeln eine etwas bessere Fertigkeit erfordert und die Gesamtdauer der Operation dadurch um etwa drei Viertel verkürzt wird. Zudem kann der spiegelreflexive ACB-Typ (Fig. 27) auch von Linkshändern problemlos verwendet werden.

### REFERENZEN

1. "Künstliche Kapseltasche und Verfahren zu seiner Fixierung", armenisches Patent Nr. AM 3047 A, erteilt am 03.10.2016.
2. "Künstliche Kapseltasche zur Befestigung einer pcIOL an der Iriswurzel", Asatur Hovsepyan, DE 20 2016 105 208.
3. "Künstliche Kapseltasche", Asatur Hovsepyan (AM), armenisches Patent Nr. AM 3302 A, erteilt am 17.06.2019.
4. "Flanged intrascleral IOL fixation with double-needle technique", Shin Yamane, MD; Ocular Surgery News U.S. Edition, October 10, 2018. URL: https://www.healio.com/ophthalmology/cataract-surgery/news/print/ocularsurgery-news/%7B317139d3-1b1a-4883-b965-c8daff4f7456%7D/flangedintrascleral-iol-fixation-with-double-needle-technigue
5. "Four-Flanged Intrascleral Intraocular Lens Fixation Technique: No Flaps, No Knots, No Glue"". Sergio Canabrava1, Ana Carolina Canêdo Domingos Lima 1, Guilherme Ribeiro; PubMed, 2020 Apr;39(4):527-528, doi: 10.1097/ICO.0000000000002185. PMID: 31658168. URL: https://pubmed.ncbi.nlm.nih.qov/31658168/

## Patentansprüche

1. Künstliche Kapseltasche zur Fixierung einer Hinterkammer-Intraokularlinse (pcIOL) (8) im menschlichen Auge bei nahezu oder vollständig fehlender Kapselunterstützung, umfassend:
- einen ringförmigen Rahmen (1) mit mehreren Halterungen (2) zur Befestigung der pcIOL (8), wobei der Rahmen (1) eine Rahmenebene definiert,
- mehrere Flügel (5), die jeweils über einen Verbindungsabschnitt (3), der sich senkrecht oder schräg zur Rahmenebene erstreckt, mit dem ringförmigen Rahmen (1) verbunden sind, wobei die Flügel (5) einen langen, drahtförmigen Abschnitt (7) aufweisen, von dem ein kurzer Fußabschnitt (6) abzweigt
**dadurch gekennzeichnet, dass**
die langen, drahtförmigen Abschnitte (7) der Flügel (5) alle in die gleiche Umfangsrichtung, also entweder in Uhrzeigerrichtung oder entgegen der Uhrzeigerrichtung, zeigen.

2. Künstliche Kapseltasche gemäß Anspruch 1, wobei die langen, drahtförmigen Abschnitte (7) der Flügel (5) bogenförmig gekrümmt sind.

3. Künstliche Kapseltasche gemäß Anspruch 1, wobei die langen, drahtförmigen Abschnitte (7) Enden (14) aufweisen, die in Umfangsrichtung betrachtet in eine erste Richtung zeigen, wohingegen die kurzen Fußabschnitte (6) in die entgegengesetzte Umfangsrichtung zeigen.

4. Künstliche Kapseltasche gemäß Anspruch 1, wobei der Rahmen (1) und die Flügel (5) im Wesentlichen in parallelen Ebenen liegen.

5. Künstliche Kapseltasche gemäß Anspruch 1, wobei am Rahmen (1) drei Flügel (5) befestigt sind.

6. Künstliche Kapseltasche gemäß Anspruch 1, wobei die Position, an der die kurzen Fußabschnitte (6) von den drahtförmigen Abschnitten (7) abzweigen, näher am Rahmen (1) als am Ende (14) der drahtförmigen Abschnitte (7) liegt.

7. Künstliche Kapseltasche zur Fixierung einer Hinterkammer-Intraokularlinse (pcIOL) (8) im menschlichen Auge bei nahezu oder vollständig fehlender Kapselunterstützung, umfassend:
- einen ringförmigen Rahmen (1) mit mehreren Halterungen (2) zur Befestigung der pcIOL (8), wobei der Rahmen (1) eine Rahmenebene definiert,
- mehrere Flügel (5), die jeweils über einen Verbindungsabschnitt (3), der sich senkrecht oder schräg zur Rahmenebene erstreckt, mit dem ringförmigen Rahmen (1) verbunden sind, wobei die Flügel (5) jeweils ein Fußende (4) aufweisen, das sich im weiteren Verlauf nach außen in zwei drahtförmige Abschnitte (7) aufteilt, die symmetrisch gegenüberliegend angeordnet sind
**dadurch gekennzeichnet, dass**
die drahtförmigen Abschnitte (7) jeweils ein freies Ende (14) aufweisen, wobei die freien Enden (14) der symmetrisch gegenüberliegenden drahtförmigen Abschnitte (7) in entgegengesetzte Richtungen zeigen; und die drahtförmigen Abschnitte (7) zwischen dem Fußende (4) und ihrem jeweiligen freien Ende (14) bogenförmig gekrümmt sind.

8. Künstliche Kapseltasche gemäß Anspruch 7, wobei die zwei drahtförmigen Abschnitte (7) eines Flügels (5) gleich lang sind.
